# EUROPEAN PATENT APPLICATION

(11) **EP 3 375 905 A1**
(43) Date of publication of application: **19.09.2018**
(21) Application number: 17189374.6
(22) Date of filing: 05.09.2017
(51) Int. Cl.: C25B 1/00, C25B 1/04, C25B 3/04, C07C 29/60, C07C 31/08, C07C 1/24, C07C 11/04, C07C 9/06

(54) **CHEMICAL REACTION SYSTEM**

(30) Priority: 16.03.2017 JP 2017051309
(71) Applicant: KABUSHIKI KAISHA TOSHIBA, Minato-ku Tokyo 105-8001 (JP)
(72) Inventor: Sugano, Yoshitsune, Tokyo, 105-8001 (JP); Tamura, Jun, Tokyo, 105-8001 (JP); Ono, Akihiko, Tokyo, 105-8001 (JP); Kitagawa, Ryota, Tokyo, 105-8001 (JP); Kudo, Yuki, Tokyo, 105-8001 (JP); Yamagiwa, Masakazu, Tokyo, 105-8001 (JP); Mikoshiba, Satoshi, Tokyo, 105-8001 (JP)
(74) Representative: Hoffmann Eitle

(57) **Abstract**

A chemical reaction system includes: an electrochemical reaction device including a reduction part to store an electrolytic solution containing carbon dioxide and to reduce the carbon dioxide and thus generate a first product in the electrolytic solution; a reactor including a reaction chamber to store a reaction solution containing the electrolytic solution from the electrochemical reaction device and to chemically react a reactant containing the first product in the reaction solution and thus generate a second product in the reaction solution; and a separation and recovery device including a separation chamber to store a product solution containing the reaction solution from the reactor and to extract and recover at least a part of the second product from the product solution.

## Description

### FIELD

Arrangements described herein generally relate to a chemical reaction system.

### BACKGROUND

In recent years, reduction in carbon dioxide being greenhouse effect gas has been demanded from viewpoints of energy problems and environmental problems. In particular, carbon dioxide reduction technologies such as artificial photosynthesis are able to directly convert carbon dioxide into fuel and chemical materials, and therefore, it is largely advantageous from a viewpoint that deletion problems of fossil fuel and global warming problems can be simultaneously solved.

As products which are obtained when carbon dioxide is reduced to other substances, for example, there can be cited carbon monoxide (CO), formic acid (HCOOH), and so on. Besides, there can be cited methanol, methane, and so on as multielectron reduction reaction products which are reduced by using more electrons. Further, two or more carbon dioxides are simultaneously reduced, and thereby, it is also possible to generate a compound having C-C bond such as ethylene and ethanol. These multielectron reduction reaction products have extremely high usefulness in contemporary society, and early actualization thereof has been demanded. However, selectivity of the multielecton reduction reaction products generated by the existing carbon dioxide reduction technology is low, and it is difficult to mainly obtain these products.

Organic compounds such as ethylene are manufactured by, for example, being separated from naphtha. Besides, it is also possible to manufacture by a technology such as a low-temperature separation. However, a lot of separation steps are required in the separation step from naphtha, and a lot of energy accompanied by high temperature and a lot of cost are required. It is also the same as for the low-temperature separation. Besides, according to these manufacturing methods, it is necessary to simultaneously treat products other than a target, and the efficiency is low from a viewpoint of obtaining only the target product. Accordingly, a more selective and energy-saving manufacturing step has been demanded.

### RELEVANT REFERENCES

### Patent Reference

Reference1: WO2014/065421
Reference 2: JPA 2004-35704
Reference 3: JPA 2000-309549
Reference 4: JPA 2014-185112
Reference 5: US 2013/0178674
Reference 6: JPA 2015-020972

### Non-patent Reference

Reference 7: M. Chia et al., Journal of the American Chemical Society. vol 133, pp. 12675-12689,2011

### BRIEF DESCRIPTION OF THE DRAWINGS

Fig. 1 is a schematic view illustrating a configuration example of a chemical reaction system.
Fig. 2 is a schematic view illustrating a configuration example of a chemical reaction system.
Fig. 3 is a schematic view illustrating a configuration example of a chemical reaction system.
Fig. 4 is a schematic view illustrating a configuration example of a chemical reaction system.
Fig. 5 is a schematic view illustrating a configuration example of a chemical reaction system.

### DETAILED DESCRIPTION

A chemical reaction system of the arrangement includes: an electrochemical reaction device including a reduction part to store an electrolytic solution containing carbon dioxide and to reduce the carbon dioxide and thus generate a first product in the electrolytic solution; a reactor including a reaction chamber to store a reaction solution containing the electrolytic solution from the electrochemical reaction device and to chemically react a reactant containing the first product in the reaction solution and thus generate a second product in the reaction solution; and a separation and recovery device including a separation chamber to store a product solution containing the reaction solution from the reactor and to extract and recover at least a part of the second product from the product solution.

Hereinafter, arrangements are described with reference to the drawings. Note that the drawings are schematic, and for example, dimensions such as a thickness, a width of each constituent element may differ from dimensions of actual constituent elements. In the arrangements, substantially the same constituent elements are denoted by the same reference signs and a description thereof will be omitted in some cases. In the description, a term of "connection" may include a meaning of indirect connection without being limited to a case of direct connection.

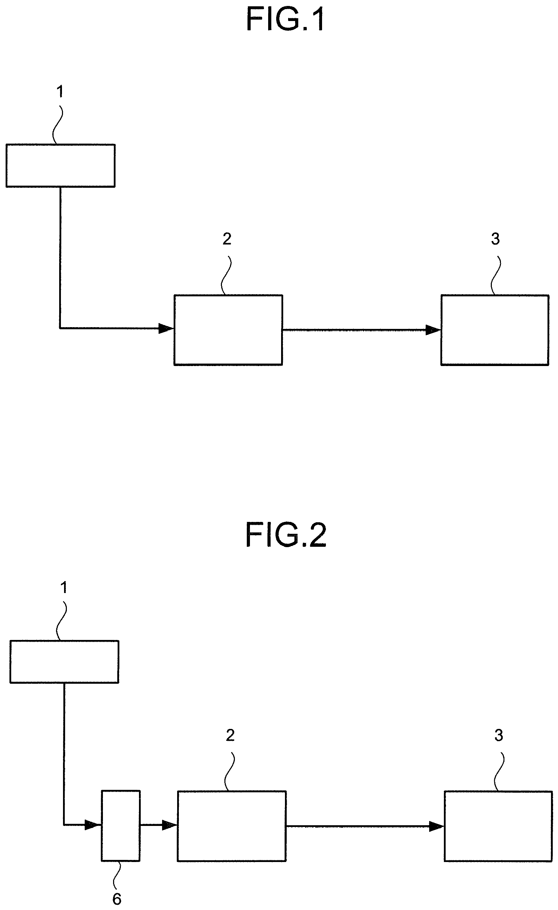

Fig. 1 to Fig. 5 are schematic views each illustrating a configuration example of a chemical reaction system. The chemical reaction system includes an electrochemical reaction device 1, a reaction device 2, and a separation and recovery device 3. Note that arrows illustrated in Fig. 1 to Fig. 5 each represent a moving direction of a substance through a flow path. Besides, description of common components among Fig. 1 to Fig. 5 can be appropriately quoted with each other.

The electrochemical reaction device 1 is able to reduce carbon dioxide to generate a product A containing a carbon compound or the like. An example of the product A includes the carbon compound or the like having two or more carbon chains such as ethylene glycol. The examples of the product A may include carbon monoxide, formic acid, ethanol, propanol, citric acid, ethylene, methane, and so on without being limited to the above. Besides, the product A may contain hydrogen. Further, the electrochemical reaction device 1 may oxidize water to generate oxygen or the like.

The reaction device 2 includes a reactor capable of chemically reacting a reactant containing the product A supplied from the electrochemical reaction device 1 through a flow path such as a pipe to generate a product B. The product A may be contained in, for example, an electrolytic solution supplied from the electrochemical reaction device 1. Besides, the reactant may contain hydrogen and oxygen supplied from the electrochemical reaction device 1 through the flow path such as the pipe. An example of the product B includes the carbon compound having two or more carbon chains such as ethanol and ethylene.

The separation and recovery device 3 includes a separation and recovery device capable of refining a product solution containing the product B supplied from the reaction device 2 through the flow path such as the pipe to extract and recover at least a part of the product B from the product solution.

The chemical reaction system may further include a buffer tank 6 which is connected between the electrochemical reaction device 1 and the reaction device 2 as illustrated in Fig. 2. The buffer tank 6 is able to store at least a part of the electrolytic solution containing the product A supplied from the electrochemical reaction device 1. The electrolytic solution which is stored for a certain period is then supplied to the reaction device 2. In the chemical reaction at the reaction device 2, it is conceivable that a proceeding speed of the chemical reaction largely varies depending on a composition ratio of each raw material. On the other hand, it is possible to adjust the composition ratio of reaction raw materials supplied to the reaction device 2 by providing the buffer tank 6. Note that the pipe connecting between the electrochemical reaction device 1 and the reaction device 2 may have a function as the buffer tank 6 instead of providing the buffer tank 6.

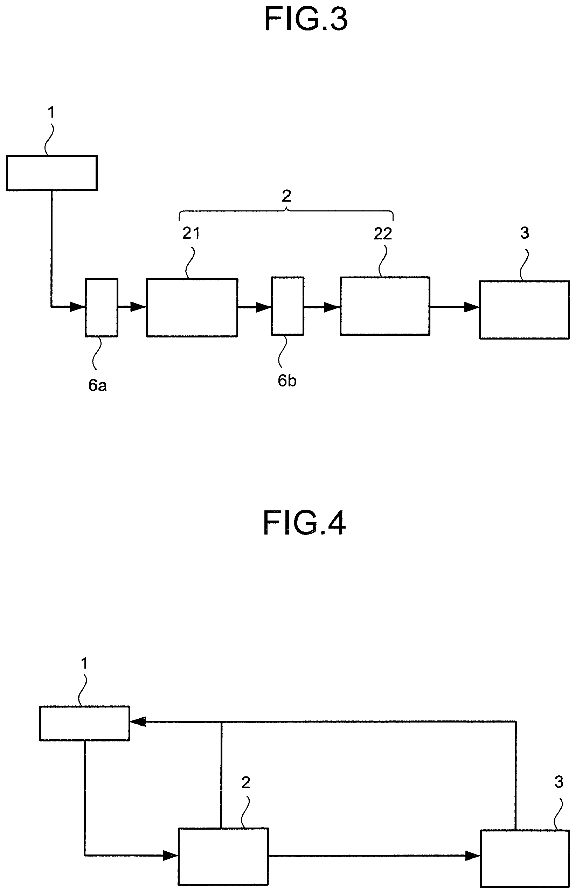

In the chemical reaction system, the reaction device 2 may include a reactor 21 and a reactor 22 as illustrated in Fig. 3. The reactor 21 and the reactor 22 are able to generate products different from one another. Besides, a buffer tank 6a connected between the electrochemical reaction device 1 and the reactor 21, and a buffer tank 6b connected between the reactor 21 and the reactor 22 may be provided as the buffer tank 6.

The chemical reaction system may supply at least a part of the substances in the reaction device 2 and the separation and recovery device 3 to the electrochemical reaction device 1 through the flow path such as the pipe as illustrated in Fig. 4.

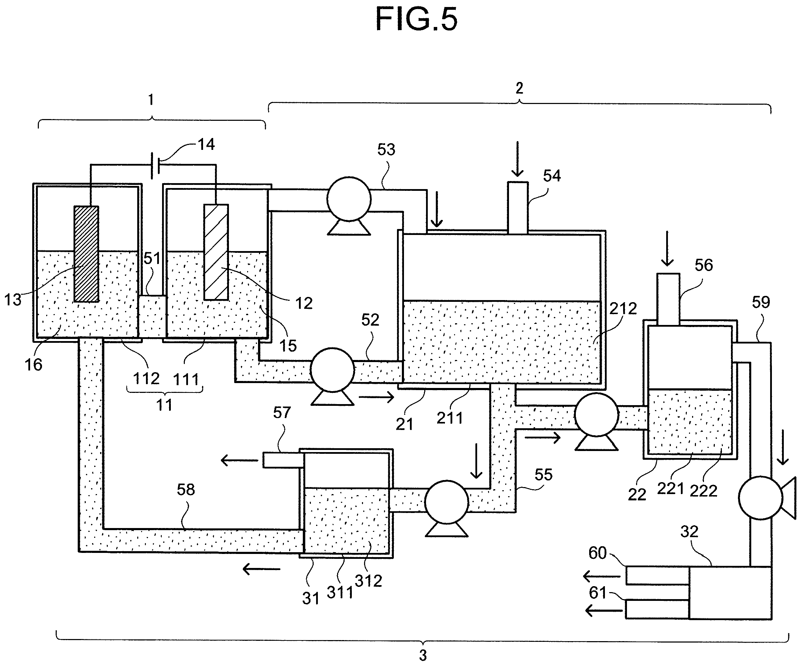

Next, a chemical reaction system illustrated in Fig. 5 is described. The electrochemical reaction device 1 illustrated in Fig. 5 includes an electrolytic solution tank 11, a cathode 12, an anode 13, and a power supply 14.

The electrolytic solution tank 11 includes a room 111 and a room 112. The room 111 is able to store an electrolytic solution 15. The room 112 is able to store an electrolytic solution 16. The room 111 and the room 112 are connected by a pipe 51. Note that an ion exchange membrane may be provided at the pipe 51.

At least one of the electrolytic solution 15 and the electrolytic solution 16 may contain a fluid containing at least one of a solid body, liquid and gas, a supercritical fluid or the like. Besides, organic compounds and inorganic compounds may be contained, an organic-inorganic hybrid compound such as an inorganic complex may be contained, and a plurality kinds of the above may be contained.

At least one of the electrolytic solution 15 and the electrolytic solution 16 may contain, for example, an aqueous solution or the like containing: alkali metal salts such as LiHCO₃, NaHCO₃, KHCO₃, CsCO₃; alkali earth metal salts, and so on. Besides, there may be contained: alcohols such as ethylene glycol, methanol, ethanol and propanol; hydrocarbons such as methane, ethylene, ethane and propylene; acids such as hydrochloric acid, nitric acid, sulfuric acid, phosphoric acid, boric acid, formic acid, acetic acid, oxalic acid, citric acid and sulfonic acid; compounds having a carbonyl group such as aldehyde and ketone, an amino group and a nitro group; compounds having an ether bond, an ester bond, an amid bond, and so on; oxygen; nitrogen; hydrogen; carbon monoxide; carbon dioxide, and so on.

As at least one of the electrolytic solution 15 and the electrolytic solution 16, there may be used, an ionic liquid which is made of salts of cations such as imidazolium ions or pyridinium ions and anions such as BF₄⁻ or PF₆⁻ and which is in a liquid state in a wide temperature range, or aqueous solutions thereof. Further, as other electrolytic solutions, there may be contained amine solutions such as ethanolamine, imidazole, pyridine, or aqueous solutions thereof. As amine, there can be cited primary amine, secondary amine, tertiary amine, or the like. These electrolytic solutions may have properties with high ion conductivity and adsorbing carbon dioxide, and have a characteristic lowering reduction energy.

As the primary amine, there can be cited methylamine, ethylamine, propylamine, butylamine, pentylamine, hexylamine, and so on. Hydrocarbons of the amine may be substituted by alcohol, halogen, and so on. As amine whose hydrocarbons are substituted, there can be cited methanolamine, ethanolamine, chloromethyl amine, and so on. Further, an unsaturated bond may exist. These hydrocarbons are also applied to the secondary amine and the tertiary amine.

As the secondary amine, there can be cited dimethylamine, diethylamine, dipropylamine, dibutylamine, dipentylamine, dihexylamine, dimethanolamine, diethanolamine, dipropanolamine, and so on. The substituted hydrocarbons may be different. This also applies to the tertiary amine. For example, as examples whose substituted hydrocarbons are different, there can be cited methylethylamine, methylpropylamine, and so on.

As the tertiary amine, there can be cited trimethylamine, triethylamine, tripropylamine, tributylamine, trihexylamine, trimethanolamine, triethanolamine, tripropanolamine, tributanolamine, tripropanolamine, triexanolamine, methyldiethylamine, methyldipropylamine, and so on.

As the cations of the ionic liquid, there can be cited 1-ethyl-3-methylimidazolium ions, 1-methyl-3-propylimidazolium ions, 1-butyl-3-methylimidazole ions, 1-methyl-3-pentylimidazolium ions, 1-hexyl-3-methylimidazolium ions, and so on.

A second place of the imidazolium ions may be substituted. As the cations whose second place of the imidazolium ions is substituted, there can be cited 1-ethyl-2,3-dimethylimidazolium ions, 1,2-dimethyl-3-propylimidazolium ions, 1-butyl-2,3-dimethylimidazolium ions, 1,2-dimethyl-3-pentylimidazolium ions, 1-hexyl-2,3-dimethylimidazolium ions, and so on.

As the pyridinium ions, there can be cited methylpyridinium, ethylpyridinium, propylpyridinium, butylpyridinium, pentylpyridinium, hexylpyridinium, and so on. In both of the imidazolium ions and the pyridinium ions, an alkyl group may be substituted, or an unsaturated bond may exist.

These imidazolium molecule, pyridinium molecule may be bonded by ethylene glycol chains. For example, there can be cited polyethylene glycol m (methylimidazolium)2 (m = 10 to 10000), and so on.

As the anions, there can be cited fluoride ions, chloride ions, bromide ions, iodide ions, BF₄⁻, PF₆⁻, CF₃COO⁻, CF₃SO₃⁻, NO₃⁻, SCN⁻, (CF₃SO₂)₃C⁻, bis(trifluoromethoxysulfonyl)imide, bis(trifluoromethoxysulfonyl)imide, bis(perfluoroethylsulfonyl)imide, bistrifluoromethanesulfonyl, and so on. Dipolar ions in which the cations and the anions of the ionic liquid are coupled by hydrocarbons may be used. Note that it may be a buffer solution such as a potassium phosphate solution.

As an organic solvent, there can be cited, for example, toluene, benzene, octane, octanol, dimethylformanide, hexane, xylene, chlorobenzene, ethyl acetate, chloroethylene, dichloroethylene, acetic acid, formalin, formic acid, acetaldehyde, tetrahydrofuran, cyclohexane, methanol, ethanol, ethylene glycol, glyoxal, acetonitrile, methyl acetate, ethyl methyl ketone, dimethyl ether, acetone, diethyl ether, chloroform, methylene chloride, dimethyl sulfoxide (DMSO), dimethyl disulfide (DMDS), or as an aprotic solvent, there can be cited hexamethylphosphoric triamide (HMPA), N,N'-dimethylpropyleneurea (DMPU), and so on. These can be each used alone or as a mixture.

The cathode 12 is disposed in the room 111, and is immersed in the electrolytic solution 15. A reduction reaction part including the cathode 12 and the room 111 is provided to reduce carbon dioxide or the like to generate the product A in the electrolytic solution 15.

The cathode 12 contains a reduction catalyst to generate the product A such as the carbon compound by the reduction reaction of carbon dioxide or the like. As the reduction catalyst, there can be cited a material lowering activation energy to reduce hydrogen ions and carbon dioxide. In other words, there can be cited a material lowering overvoltage when hydrogen and the carbon compound are generated by the reduction reaction of hydrogen ions and carbon dioxide. For example, a metal material and a carbon material can be used. As the metal material, for example, there can be used metals such as platinum and nickel, or an alloy containing these metals in case of hydrogen. In the reduction reaction of carbon dioxide, there can be used metals such as gold, aluminum, copper, silver, zinc, gallium, cadmium, indium, tin, thallium, lead, platinum, palladium, nickel, or an alloy containing these metals. As the carbon material, there can be used, for example, graphene, carbon nanotube (CNT), fullerene, ketjen black, and so on. The reduction catalyst is not limited thereto, and there can be also used, for example, metal complexes such as an Ru complex and an Re complex, organic molecules having an amine skeleton, an imidazole skeleton, a pyridine skeleton, and the like, and exerting activity for the carbon dioxide reduction as the reduction catalyst. Besides, a plurality of materials may be mixed.

The anode 13 is disposed in the room 112, and is immersed in the electrolytic solution 16. An oxidation reaction part including the anode 13 and the room 112 is provided to oxidize water or the like to generate oxygen or the like.

The anode 13 contains an oxidation catalyst to generate a product such as hydrogen by the oxidation reaction of water or the like. As the oxidation catalyst, there can be cited a material lowering activation energy to oxidize water. In other words, there can be cited a material lowering overvoltage when oxygen and hydrogen ions are generated by the oxidation reaction of water. For example, there can be cited iridium, ruthenium, indium, tin, nickel, iron, platinum, cobalt, manganese, or the like. Besides, as the oxidation catalyst, there can be used binary metal oxides, ternary metal oxides, quaternary metal oxides, and so on. As the binary metal oxides, there can be cited, for example, manganese oxide (Mn-O), iridium oxide (Ir-O), nickel oxide (Ni-O), cobalt oxide (Co-O), iron oxide (Fe-O), tin oxide (Sn-O), indium oxide (In-O), ruthenium oxide (Ru-O), and so on. As the ternary metal oxides, there can be cited, for example, Ni-Co-O, La-Co-O, Ni-La-O, Sr-Fe-O, and so on. As the quaternary metal oxides, there can be cited, for example, Pb-Ru-Ir-O, La-Sr-Co-O, and so on. Note that the oxidation catalysts are not limited thereto, and there can also be used the metal complexes such as the Ru complex and an Fe complex as the oxidation catalyst. Besides, a plurality of materials may be mixed.

Examples of products generated by an oxidation reduction reaction may contain by-products such as a reduced product of a compound which is added at the oxidation reduction reaction time. The examples of the products generated by the oxidation reduction reaction may contain compounds generated accompanied by the reduction reaction of carbon dioxide such as sacrificial reagent such as ethylenediaminetetraacetic acid, triethanolamine and ascorbic acid. The by-product and the compound generated accompanied by the reduction reaction may be contained in at least one of the electrolytic solution 15 and the electrolytic solution 16.

The power supply 14 is electrically connected to the cathode 12 and the anode 13. For example, wirings may be used for the connections between the power supply 14 and the cathode 12, and between the power supply 14 and the anode 13. The reduction reaction by the cathode 12 and the oxidation reaction by the anode 13 are carried out by using electric energy supplied from the power supply 14. The power supply 14 includes a power supply device such as a photoelectric conversion element, a system power supply, a storage battery, or a conversion part which converts renewable energy such as wind power, water power, geothermal power or tidal power into electric energy. For example, the photoelectric conversion element has a function to perform charge separation by using light energy such as irradiated sunlight. Examples of the photoelectric conversion element include a pin junction type solar cell, a pn junction type solar cell, an amorphous silicon solar cell, a multijunction solar cell, a single crystal silicon solar cell, a polycrystalline silicon solar cell, a dye sensitized solar cell, an organic thin-film solar cell, and so on. Besides, the photoelectric conversion element may be stacked on the cathode 12 and the anode 13 in the electrolytic solution tank 11.

The reaction device 2 includes the reactor 21 and the reactor 22. The reactor 21 and the reactor 22 may each generate the product by different chemical reactions from one another. The reaction device 2 may not include the reactor 22 as illustrated in Fig. 1, Fig. 2, and so on.

The reactor 21 includes a reaction chamber 211. The reaction chamber 211 is able to store a reaction solution 212 containing substances which are contained in at least one of the electrolytic solution 15 and the electrolytic solution 16 supplied from the electrochemical reaction device 1 through a pipe 52. Accordingly, the reaction solution 212 contains the product A. Besides, the reaction chamber 211 is able to store gas such as hydrogen and oxygen generated by the oxidation reduction reaction supplied from the electrochemical reaction device 1 through the pipe 52. The reactor 21 chemically reacts reactants containing the product A in the reaction solution 212 by using a catalyst supplied to the reaction chamber 211 through a pipe 54, and is able to generate a product B1 as the product B. At this time, it is preferable that an inside of the reactor 21 is pressurized and heated by a heater, a pressurizer, and so on. The reactants may contain at least one of hydrogen and oxygen. An example of the product B1 includes ethanol or the like. The catalyst may be a homogeneous catalyst or a heterogeneous catalyst. Besides, when it is the heterogeneous catalyst, a form of the catalyst may be different such as powder, aggregate, thin-film, and so on according to the reaction. Note that it is not necessarily required to supply the catalyst to the reaction chamber 211.

The reactor 22 includes a reaction chamber 221. The reaction chamber 221 is able to store a reaction solution 222 containing substances contained in the reaction solution 212 supplied from the reactor 21 through a pipe 55. Accordingly, the reaction solution 222 contains the product B 1. The reactor 22 chemically reacts the product B1 by using a catalyst supplied to the reaction chamber 221 through a pipe 56, and is able to generate a product B2 as the product B. At this time, it is preferable that an inside of the reactor 22 is pressurized and heated by a heater, a pressurizer, and so on. The product B2 contains the carbon compound such as ethylene. The catalyst may be a homogeneous catalyst or a heterogeneous catalyst. Besides, when it is the heterogeneous catalyst, a form of the catalyst may be different such as powder, aggregate, thin-film, and so on according to the reaction. Note that it is not necessarily required to supply the catalyst to the reaction chamber 221.

Examples of the reactor 21 and the reactor 22 include various types of reactors, and a reactor suitable for a kind of the chemical reaction may be used. As the kind of the chemical reaction, there can be cited, for example, a substitution reaction, an addition reaction, an elimination reaction, a rearrangement reaction, and so on. A required reactor differs depending on kinds of operations of the chemical reaction from among a batch operation, a semi-batch operation, a continuous operation, and so on. As a tank type reactor, for example, there can be cited a batch reactor, a continuous stirred tank reactor, and so on. As a tubular reactor (piston flow reactor), for example, there can be cited a single tubular reactor, a multi-tubular reactor, and so on. Besides, it is possible to use these reactors in combination.

As the chemical reaction by the reactor 21, for example, there is a reaction where ethylene glycol is converted into ethanol. When the electrochemical reaction device 1 generates ethylene glycol, hydrogen being the by-product and the electrolytic solution are supplied to the reactor 21 through the pipe 52 and the pipe 53, a catalyst based on platinum, rhenium or rhodium is added to be heated to approximately 120°C to 180°C, to be reacted at 10 atmosphere, and thereby, it is possible to obtain ethanol in extremely high yield.

At this time, it is possible to supply the electrolytic solution together with ethylene glycol being the product as a liquid phase from the electrochemical reaction device 1 to the reaction device 2, and to supply hydrogen being the by-product as a gas phase from the electrochemical reaction device 1 to the reaction device 2. Besides, the pipe 53 may not be provided as long as the electrochemical reaction device 1 has a function to extract in a gas-liquid mixed state (for example, in case when it is possible to push out both gas and liquid such as a piston).

It is possible to simplify an operation at the reaction device 2 by heating an inside of the pipe 53 beforehand by a heater or the like to increase a temperature of the liquid up to a temperature necessary for the reaction. Besides, impurities in the reaction solution 212 may be removed by a desalination process or the like according to need.

When the product A supplied from the electrochemical reaction device 1 is used at the reaction device 2, a material having an adsorption function which attracts the product A may be used. The product is thereby locally concentrated, and when a catalytic reaction or the like is used, it is possible to accelerate a reaction rate and to increase a production amount.

As the chemical reaction by the reactor 22, there can be cited the chemical reaction generating ethylene in the extremely high yield by adding aluminum oxide to a solution containing ethanol and water, and heating and pressurizing the solution. At this time, the water used at the reactor 21 is directly used, and thereby, the operation can be simplified.

As another example of the chemical reaction by the reactor 22, there can be cited the chemical reaction converting ethanol generated by the reactor 21 into ethane with high selectivity under existence of water, palladium nitrate, and hydrogen. At this time, it is possible to use hydrogen and water which are already used at the electrochemical reaction device 1 or the reactor 21 for the reaction. As stated above, it is possible to generate the product by using a compound which cannot be reduced at the electrochemical reaction device 1, by using hydrogen being the by-product obtained at the electrochemical reaction time and water during reaction.

As other products, there can be cited alcohols such as methanol and ethanol, aldehydes such as formaldehyde and acetaldehyde, hydrocarbons such as methane, ethylene, ethane and propylene, acids such as formic acid, acetic acid, oxalic acid, citric acid and sulfonic acid, compounds having the amino group and the nitro group, compounds having the ether bond, the ester bond, the amid bond, an aromatic ring, a heterocyclic ring, and so on.

The separation and recovery device 3 includes a separation and recovery device 31 and a separation and recovery device 32. Note that the separation and recovery device 32 is not necessarily included.

The separation and recovery device 31 includes a separation chamber 311. The separation chamber 311 is able to store a product solution 312 containing substances contained in the reaction solution 212 supplied from the reactor 21 through a pipe 55. The product solution 312 contains, for example, the product B1. The separation and recovery device 31 is able to extract and recover the product B1 from the product solution 312 containing the product B1 by, for example, azeotropic separation or the like. It is preferable to carry out three-components azeotrope or the like by adding a third component so as to enable higher precision extraction. As the third component, pentane or the like can be cited. At least a part of the product B1 separated by the separation and recovery device 31 is flowed out of the separation and recovery device 31 through a pipe 57, and thereby it can be recovered. Further, the remainder of the product solution 312 except at least a part of the product B1 may be supplied from the separation and recovery device 31 to the room 112 through a pipe 58.

The separation and recovery device 32 is able to extract and recover the carbon compound from the product B2 containing the carbon compound such as ethylene supplied from the reactor 22 through a pipe 59. For example, it is possible to separate the carbon compound by membrane separation or the like. At least a part of the carbon compound such as ethylene separated by the separation and recovery device 32 is flowed out of the separation and recovery device 32 through a pipe 60, and the remainder of the product B2 except at least a part of the carbon compound is flowed out of the separation and recovery device 32 through a pipe 61.

A configuration of each of the separation and recovery device 31 and the separation and recovery device 32 is not limited to the one illustrated in Fig. 5, and a separation and recovery device capable of applying various separation methods for a separation and recovery target may be used. Besides, as the separation method, there can be used at least one of gas absorption, adsorption, radiation, extraction, chromatography, drying, condensation, partial condensation, evaporation, distillation, rectification, crystallization, sublimation, partial dissolution, dialysis, filtration, electroosmosis, electrophoretic migration, centrifugal separation and sedimentation.

A residue generated by the reaction device 2 and the separation and recovery device 3 may be reused by resupplying to at least one of the electrochemical reaction device 1, the reaction device 2, or the separation and recovery device 3.

Shapes of the pipes 51 to 61 are not particularly limited as long as it is the shape capable of transporting each substance. At least one of the pipes 51 to 61 may have a function of partially purging to outside the system. A moving direction, a flow rate, a pressure, and so on of the substance flowing in at least one of the pipes 51 to 61 may be regulated by a pump. For example, physical properties such as a temperature of fluid flowing in at least one of the pipes 51 to 61 are monitored by a monitor circuit or the like to control a state of the fluid.

An inside of at least one of the pipes 51 to 61 may be heated or cooled by at least one of a heater and a cooler. It is thereby possible to reflux the electrolytic solution and gas flowing out of the electrochemical reaction device 1 to the electrochemical reaction device 1. It is thereby possible to increase reactivity by controlling an operation temperature or the like of the electrochemical reaction device 1.

In the chemical reaction system of the arrangement, since it is possible to proceed to a next step while keeping the product generated by the electrochemical reaction device and the reaction device existing in a solution as it is, the reaction can be simplified. A manufacturing process of organic compounds at conventional petroleum plants or the like requires high temperature and a lot of separation processes, and therefore, there are problems of scale adjustment and enormous energy input amount, and so on. On the other hand, in the manufacturing process of the organic compounds in the chemical reaction system of the arrangement, the compounds are manufactured by passing through a selective substance conversion step. Accordingly, it is possible to apply minimum and optimized reaction process and separation and recovery process according to a target product, and it is excellent in viewpoints of cost and the energy input amount.

While certain arrangements have been described, these arrangements have been presented by way of example only, and are not intended to limit the scope of the claims. Indeed, the magnet described herein may be embodied in a variety of other forms; furthermore, various omissions, substitutions and changes in the form of the magnet described herein may be made.

### (Numbered Clauses relating to the arrangements)

1. A chemical reaction system, comprising:
   an electrochemical reaction device including a reduction part to store an electrolytic solution containing carbon dioxide and to reduce the carbon dioxide and thus generate a first product in the electrolytic solution;
   a reactor including a reaction chamber to store a reaction solution containing the electrolytic solution from the electrochemical reaction device and to chemically react a reactant containing the first product in the reaction solution and thus generate a second product in the reaction solution; and
   a separation and recovery device including a separation chamber to store a product solution containing the reaction solution from the reactor and to extract and recover at least a part of the second product from the product solution.
2. The system according to clause 1,
   wherein the first product contains hydrogen, and
   wherein the reactant further contains the hydrogen from the electrochemical reaction device.
3. The system according to clause 1 or clause 2,
   wherein the first product contains a first carbon compound having two or more carbon chains.
4. The system according to clause 3,
   wherein the first carbon compound contains ethylene glycol.
5. The system according to any one of clauses 1 to 4,
   wherein the second product contains a second carbon compound having two or more carbon chains.
6. The system according to clause 5,
   wherein the second carbon compound contains ethanol.
7. The system according to any one of clauses 1 to , further comprising:
   a second reactor including a second reaction chamber to store a second reaction solution containing the reaction solution from the reactor and to chemically react a second reactant containing the second product in the second reaction solution and thus generate a third product containing a third carbon compound; and
   a second separation and recovery device to extract at least a part of the third carbon compound from the third product supplied from the second reactor.
8. The system according to clause 7,
   wherein the third carbon compound contains ethylene.
9. The system according to any one of clauses 1 to 8,
   wherein the electrochemical reaction device further includes an oxidation part to store a second electrolytic solution containing water and to oxidize the water and thus generate oxygen.
10. The system according to clause 9, further comprising:
   a flow path through which a remainder of the product solution except at least a part of the extracted second product is supplied from the separation and recovery device to the oxidation part.
11. The system according to clause 9 or clause 10,
   wherein the reduction part includes a first room to store the electrolytic solution and a cathode inside the first room, and
   wherein the oxidation part includes a second room to store the second electrolytic solution and an anode inside the second room.
12. The system according to any one of clauses 1 to 11, further comprising:
   a buffer tank connecting between the electrochemical reaction device and the reactor and configured to store at least a part of the electrolytic solution to be supplied from the electrochemical reaction device to the reactor.

## Claims

1. A chemical reaction system, comprising:
an electrochemical reaction device including a reduction part to store an electrolytic solution containing carbon dioxide and to reduce the carbon dioxide and thus generate a first product in the electrolytic solution;
a reactor including a reaction chamber to store a reaction solution containing the electrolytic solution from the electrochemical reaction device and to chemically react a reactant containing the first product in the reaction solution and thus generate a second product in the reaction solution; and
a separation and recovery device including a separation chamber to store a product solution containing the reaction solution from the reactor and to extract and recover at least a part of the second product from the product solution.

2. The system according to claim 1,
wherein the first product contains hydrogen, and
wherein the reactant further contains the hydrogen from the electrochemical reaction device.

3. The system according to claim 1 or claim 2,
wherein the first product contains a first carbon compound having two or more carbon chains.

4. The system according to claim 3,
wherein the first carbon compound contains ethylene glycol.

5. The system according to any one of claims 1 to 4,
wherein the second product contains a second carbon compound having two or more carbon chains.

6. The system according to claim 5,
wherein the second carbon compound contains ethanol.

7. The system according to claim 1, further comprising:
a second reactor including a second reaction chamber to store a second reaction solution containing the reaction solution from the reactor and to chemically react a second reactant containing the second product in the second reaction solution and thus generate a third product containing a third carbon compound; and
a second separation and recovery device to extract at least a part of the third carbon compound from the third product supplied from the second reactor.

8. The system according to claim 7,
wherein the third carbon compound contains ethylene.

9. The system according to any one of claims 1 to 8,
wherein the electrochemical reaction device further includes an oxidation part to store a second electrolytic solution containing water and to oxidize the water and thus generate oxygen.

10. The system according to claim 9, further comprising:
a flow path through which a remainder of the product solution except at least a part of the extracted second product is supplied from the separation and recovery device to the oxidation part.

11. The system according to claim 9 or claim 10,
wherein the reduction part includes a first room to store the electrolytic solution and a cathode inside the first room, and
wherein the oxidation part includes a second room to store the second electrolytic solution and an anode inside the second room.

12. The system according to any one of claims 1 to 11, further comprising:
a buffer tank connecting between the electrochemical reaction device and the reactor and configured to store at least a part of the electrolytic solution to be supplied from the electrochemical reaction device to the reactor.
